# EUROPEAN PATENT APPLICATION

(11) **EP 4 778 521 A1**
(43) Date of publication of application: **22.07.2026**
(21) Application number: 25305047.0
(22) Date of filing: 15.01.2025
(51) Int. Cl.: A61K 31/495, A61P 25/00, A61P 25/22

(54) **USE OF DOPAMINE D3 PARTIAL AGONISTS FOR TREATING COMPULSIVE REPETITIVE DISORDERS**

(71) Applicant: SOCIETE CIVILE BIOPROJET, 75003 Paris (FR)
(72) Inventor: The designation of the inventor has not yet been filed
(74) Representative: Lavoix

(57) **Abstract**

The present invention concerns the use of D3 partial agonists for treating disorders such as obsessive compulsive disorder or tics, including Gilles de la Tourette syndrome.

## Description

The present invention concerns the field of compulsive repetitive disorders, with or without obsessions, such as obsessive-compulsive disorders and tics disorders.

Obsessive-compulsive disorder (OCD) is characterized by obsessions i.e., recurring, persistent, unwanted, anxiety-provoking, intrusive ideas, images, or urges, and compulsions (also called rituals) that are certain repetitive actions or mental acts that the patients feel driven to repeatedly perform to try to lessen or prevent the anxiety caused by the obsessions.

Other obsessive-compulsive related disorders primarily involve recurring body-focused repetitive behaviours and repeated attempts to stop the behaviour. These include hoarding disorder, body dysmorphic disorder, trichotillomania (hair-pulling disorder), excoriation (skin-picking) disorder, and olfactory reference disorder.

A type of cognitive-behavioural therapy (CBT) known as exposure and response (ritual) prevention (ERP) is the first-line therapy for OCD. However, the typical course of CBT treatment for OCD requires several months and substantial involvement of the patient and its relatives.

Medications known as selective serotonin-reuptake inhibitors (SSRIs) are the other first-line treatment for OCD. However, effective SSRI doses for OCD are much higher than those used to treat depression. They can have a variety of side effects. These include nausea, nervousness, and sleep disorders. Further, it often takes six to twelve weeks for improvement in OCD symptoms to occur. An SSRI/SRI should be tried for at least 12 weeks, reaching a high enough dose during that time and being sure to take the medication every day. Nevertheless, about half of the patients show no significant improvement after an adequate trial with SSRIs.

Recent studies suggest that transcranial magnetic stimulation (TMS) can be effective for OCD. Deep brain stimulation (DBS), which involves an implanted device in the brain, has data to support efficacy, but it is invasive and complex to manage, and there are limited providers and hospital systems trained to offer this treatment and provide the long-term support needed by patients who are treated with DBS.

Recently, dopamine has been involved in OCD neuropathology (Conti et al. Human Psychopharmacol. 2024, 39(3):e2893). Antipsychotics highly effective as dopamine D2 antagonists, such as risperidone, aripiprazole and haloperidol are useful medications. Studies have investigated the addition of dopamine D2 antagonists such as olanzapine, quetiapine and risperidone to the SSRI medication (Koo et al, Expert Rev Neurother 2010, 10(2), 275-290).

These atypical antipsychotics are however unselective in that they block several receptors, let alone several if not all dopamine receptor subtypes.

Additionally, Chen et al Behav. Brain. Funct. (2021), 17:4 evidenced that OCD symptoms are mediated by distinct brain regions. As OCD is a highly heterogenous disease, the authors suggest an unpredictability between treatments and efficacy on behavioural traits in OCD patients.

Koo et al also report that selective full D2 and D3 agonists such as quinpirole induce the symptoms associated with OCD. They also report that dopamine receptor antagonists may aggravate the OCD symptoms.

Nevertheless, no clear relation between OCD and dopamine receptors, let alone the dopamine receptor subtypes D1-D5 seems to have been established. The main challenge lies in the complexity of the dopaminergic system and its role in OCD.

D3 receptors are part of a complex neural network involving different signalling pathways and multiple brain regions. In particular,
Tics are other compulsive repetitive disorders. Contrary to OCD they are not obsessive. They are typically rapid, purposeless, repetitive but not rhythmic, involuntary movements (muscle or motor tics) or involuntary, abrupt, often repetitive sounds and/or words (vocal tics). Among tics, Gilles de la Tourette syndrome (also called Tourette syndrome) is diagnosed when both motor and vocal tics have persisted for more than a year

The mechanism for tics has not been elucidated. Comprehensive behavioural intervention (BIT) may help, and medications may be necessary to stop resistant tics. However, such medications include antipsychotic medications which typically involve side effects and are not favoured in the long term for treating children which are mainly affected by tics. Tics disorders thus also require alternative treatments, with lesser side effects.

Further, one of the targets is dopamine D2 receptors, but the mechanism is still unclear: either dopamine D2 antagonists such risperidone or dopamine D2 agonists such as aripiprazole are prescribed. In addition, these compounds are not selective at the dopamine D2 receptors and interact with several other non-dopaminergic receptors as mentioned above.

Koo et al report that antidopaminergic agents such as haloperidol, risperidone and pimozide may be effective in treating tic symptoms. However, they also report that dopamine receptor antagonists may aggravate the tic symptoms.

The nexus between OCD or tics symptoms and the one hand and the dopamine ligands, let alone the D2 or D3 ligands on the other hand is thus not clearly elucidated.

WO 2007/148208 discloses carbonylated (aza)cyclohexane derivatives as dopamine D3 receptor ligands.

US 2010/0069416 discloses azabicyclo compounds acting as dopamine ligands that may be possibly useful for treating a variety of dopamine associated disorders, including OCD and related disorders such as Tourette syndrome, although no result of activity on these indications is given and the agonist or antagonist activity of the compounds is not determined.

WO 2021/224235 discloses the use of compound BP1.4979, a D3 partial agonist and D2 antagonist to treat selected disorders for which a D3 full agonistic activity is generally desired, such as restless legs syndrome, binge eating and essential tremor. It is totally silent regarding disorders requiring treatment with a dopamine antagonist, such as OCD and tics.

There is therefore a need to provide alternative non-invasive and more effective treatments for treating OCD and/or tics.

It is also desirable to provide more selective medications acting more specifically on dopamine receptors subtypes with a view to involve less side effects.

Also, it is desirable to propose medications that require lower dosage and/or shorter administration duration to reach effectiveness.

According to an object, the present invention is directed to compound BP1.4979, of formula: 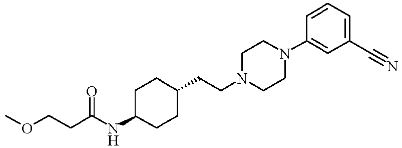
*N*-(4-{2-[4-(3-Cyanophenyl)piperazin-1-yl]ethyl}cyclohexyl)-3-methoxypropanamide or a pharmaceutically acceptable salt thereof, or its hydrates, or hydrated salts, or the polymorphic, crystalline structures thereof;
for use for treating or preventing a repetitive compulsive disorder chosen from obsessive compulsive disorder (OCD) and related disorders and tic disorders in a patient.

According to an object, the present invention is directed to a compound of the formula (A): 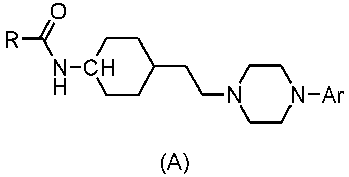
for use for treating or preventing a repetitive compulsive disorder chosen from obsessive compulsive disorder (OCD) and related disorders and tic disorders in a patient,
wherein in Formula (A):
   R is chosen from the group consisting in:
   cyanoalkyl; monohalogenoalkyl; polyhalogenoalkyl; monohalogenocycloalkyl; polyhalogeno-cycloalkyl; alkoxy; monohalogenoalkoxy; polyhalogenoalkoxy; alkoxyalkyl; monohalogenoalkoxyalkyl; polyhalogenoalkoxyalkyl; alkoxyalkoxyalkyl; monohalogenoalkoxyalkoxyalkyl; polyhalogenoalkoxyalkoxyalkyl; monohalogenocyanoalkyl; polyhalogenocyanoalkyl; cyanocycloalkyl; aryloxy; aryloxyalkyl; arylalkoxy; cycloalkenyl; cycloalkenylalkyl; cycloalkenyl fused with benzene; alkynyl; dialkylaminoalkyl; hydroxyalkyl; polyhalogenodialkylaminoalkyl; arylaminoalkyl; alkoxyalkylamino; alkoxy(alkyl)amino; cyanoalkylamino; alkylcarbonylalkyl; acylaminoalkyl; aminocarbonylalkyl; alkylsulfanylalkyl; alkylsulfinylalkyl; alkylsulfonylalkyl; alkylcarbonyl; mono- or polyhalogenoaryl; mono- or polyhalogenoaryloxyalkyl; and 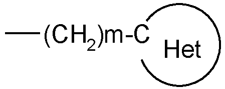 where 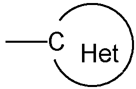 is a non aromatic heterocycle optionally fused with aryl or optionally substituted with one or more acyl, alkyl or halogen;
   m being an integer from 0 to 4,
   Ar represents an aryl; an heteroaryl or an aryl fused with a cycloalkyl or an heterocycle; Ar being optionally substituted with one or more alkyl; alkenyl; alkynyl; cyano; halogeno; alkoxy; monohalogenoalkoxy; polyhalogenoalkoxy; alkoxyalkyl; dialkylamino; non aromatic heterocyclyl attached by a nitrogen; alkylsulfanyl; alkylsulfinyl; alkylsulfonyl; monohalogenoalkylsulfanyl; monohalogenoalkylsulfinyl; monohalogenoalkylsulfonyl; polyhalogenoalkylsulfanyl; polyhalogenoalkylsulfinyl; polyhalogenoalkylsulfonyl; heteroaryl; aryl; aralkyl; aryloxy; alkoxy-carbonylamino; acyl; acylamino; aminocarbonyl; monoalkylaminocarbonyl; dialkylaminocarbonyl; alkylsulfonylamino; monohalogenoalkyl; polyhalogenoalkyl; hydroxyl; hydroxyalkyl; oxoalkyl;
   or their pharmaceutically acceptable salts, hydrates, or hydrated salts, or the polymorphic, crystalline structures of these compounds or their optical isomers, racemates, diastereomers or enantiomers.

According to an embodiment, in Formula (A):
when Ar represents a phenyl fused with a cycloalkyl and R is 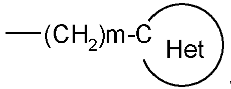 with Het being a non aromatic heterocycle and m=0, then the heterocycle is linked to the carbonyl by a carbon atom.

According to an embodiment, R is chosen from the group consisting in cyanoalkyl; polyhalogenoalkyl; alkoxy; alkoxyalkyl; polyhalogenocyanoalkyl; cyanocycloalkyl; aryloxyalkyl; arylalkoxy; cycloalkenyl; cycloalkenylalkyl; cycloalkenyl fused with benzene; dialkylaminoalkyl; hydroxyalkyl; alkylcarbonylalkyl; acylaminoalkyl; aminocarbonylalkyl; alkylsulfinylalkyl; alkylsulfonylalkyl; alkylcarbonyl; and 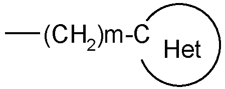 where 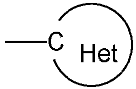 is a non aromatic heterocycle optionally fused with aryl or optionally substituted with one or more acyl, alkyl or halogen;
m being an integer from 0 to 4.

According to an embodiment, Ar represents an aryl, such as phenyl.

According to an embodiment, Ar represents an aryl optionally fused with a cycloalkyl or with an heterocycle; and/or Ar being optionally substituted with one or more alkyl; cyano; halogeno; alkoxy; polyhalogenoalkoxy; alkoxyalkyl; dialkylamino; alkylsulfanyl; alkylsulfonyl; polyhalogenoalkylsulfanyl; heteroaryl; aryloxy; alkoxy-carbonylamino; acyl; aminocarbonyl; alkylsulfonylamino; polyhalogenoalkyl; hydroxyl; hydroxyalkyl.

According to an embodiment, Ar is substituted with one or more alkyl, polyhalogenoalkyl, halogen, cyano.

According to an embodiment, said compound of formula (A) is compound BP1.4979.

Compounds of formula (A) above, such as BP1.4979 may be prepared by application or adaptation of the procedures disclosed in WO2007/148 208.

As used hereabove or hereafter:
"Acyl" means an H-CO- or alkyl-CO- group wherein the alkyl group is as herein described. Preferred acyls contain a lower alkyl. Exemplary acyl groups include formyl, acetyl, propanoyl, 2-methylpropanoyl, butanoyl and palmitoyl.
"Acylamino" is an acyl-NH- group wherein acyl is as defined herein.
"Acylaminoalkyl" means an acyl-NH-alkyl wherein acyl and alkyl are as defined herein.
"Alkenyl" means an aliphatic hydrocarbon group containing a carbon-carbon double bond and which may be straight or branched having 2 to 15 carbon atoms in the chain. Preferred alkenyl groups have 2 to 12 carbon atoms in the chain; and more preferably about 2 to 4 carbon atoms in the chain. Exemplary alkenyl groups include ethenyl, propenyl, n-butenyl, i-butenyl, 3-methylbut-2-enyl, n-pentenyl, heptenyl, octenyl, nonenyl, decenyl.
"Alkoxy" means an alkyl-O- group wherein the alkyl group is as herein described. Exemplary alkoxy groups include methoxy, ethoxy, n-propoxy, i-propoxy, n-butoxy and heptoxy.
"Alkoxyalkyl" means an alkyl-O-alkyl- group wherein the alkyl groups are independent as herein described. Exemplary alkoxy groups include methoxyethyl, ethoxymethyl, n-butoxymethyl and cyclopentylmethyloxyethyl.
"Alkoxyalkoxyalkyl" means an alkyl-O-alkyl-O-alkyl- group wherein the alkyl groups independently are as defined above.
"Alkoxyalkylamino" means an alkyl-O-alkyl-NH- wherein alkyl is as defined herein.
"Alkoxy(alkyl)amino" means an alkyl-O-N(alkyl)- wherein alkyl is as defined herein.
"Alkyl" means an aliphatic hydrocarbon group which may be straight or branched having 1 to 20 carbon atoms in the chain. Preferred alkyl groups have 1 to 12 carbon atoms in the chain. Branched means that one or more lower alkyl groups such as methyl, ethyl or propyl are attached to a linear alkyl chain. Exemplary alkyl groups include methyl, ethyl, *n-*propyl, i-propyl, n-butyl, t-butyl, n-pentyl, 3-pentyl, octyl, nonyl, decyl.
Alkyl groups may be substituted with a cyano group ("Cyanoalkyl"), a hydroxyl group ("Hydroxyalkyl"), a halogeno group ("Monohalogenoalkyl") or more ("Polyhalogenoalkyl").
"Alkylamino" means an alkyl-NH- group wherein the alkyl group is as herein described.
"Alkylcarbonylalkyl" means an alkyl-CO-alkyl- wherein alkyl are independently as defined herein.
"Alkylsulfanyl" means an alkyl-S- group wherein the alkyl group is as herein described.
"Alkylsulfanylalkyl" means an alkyl-S-alkyl- group wherein the alkyl groups are independently as herein described.
"Alkylsulfinyl" means an alkyl-SO- group wherein the alkyl group is as defined herein. Preferred groups are those wherein the alkyl group is lower alkyl.
"Alkylsulfinylalkyl" means an alkyl-SO-alkyl- group wherein the alkyl groups are independently as defined above. Preferred groups are those wherein the alkyl group is lower alkyl.
"Alkylsulfonyl" means an alkyl-SOz- group wherein the alkyl group is as defined herein. Preferred groups are those wherein the alkyl group is lower alkyl.
"Alkylsulfonylalkyl" means an alkyl-SO₂-alkyl- group wherein the alkyl groups are independently as defined herein. Preferred groups are those wherein the alkyl group is lower alkyl.
"Alkylsulfonylamino" means an alkyl-SO₂-NH- wherein alkyl is as defined herein.
"Alkynyl" means an aliphatic hydrocarbon group containing a carbon-carbon triple bond and which may be straight or branched having 2 to 15 carbon atoms in the chain. Preferred alkynyl groups have 2 to 12 carbon atoms in the chain; and more preferably 2 to 4 carbon atoms in the chain. Exemplary alkynyl groups include ethynyl, propynyl, n-butynyl, 2-butynyl, 3-methylbutynyl, n-pentynyl, heptynyl, octynyl and decynyl.
"Aminocarbonylalkyl" means an NH₂-CO-alkyl- wherein alkyl is as defined herein.
"Aralkyl" means an aryl-alkyl- group wherein the aryl and alkyl are as herein described. Preferred aralkyls contain a lower alkyl moiety. Exemplary aralkyl groups include benzyl, 2-phenethyl and naphthylmethyl.
"Aryl" means an aromatic monocyclic or multicyclic hydrocarbon ring system of 6 to 14 carbon atoms, preferably of 6 to 10 carbon atoms. Exemplary aryl groups include phenyl or naphthyl.
"Arylaminoalkyl" means an aryl-NH-alkyl- wherein aryl and alkyl are as defined herein.
"Arylalkoxy" means an aryl-alkyl-O- group wherein the aryl or alkyl groups are as herein described.
"Aryloxy" means an aryl-O- group wherein the aryl group is as defined herein. Exemplary groups include phenoxy and 2-naphthyloxy.
"Aryloxyalkyl" means an aryl-O-alkyl- group wherein the aryl or alkyl groups are as herein described. An exemplary aryloxyalkyl group is phenoxypropyl.
"Cycloalkenyl" means a non-aromatic mono- or multicyclic ring system of about 3 to about 10 carbon atoms, preferably of about 5 to about 10 carbon atoms, and which contains at least one carbon-carbon double bond. Preferred ring sizes of rings of the ring system include about 5 to about 6 ring atoms. Exemplary monocyclic cycloalkenyl includes cyclopentenyl, cyclohexenyl, cycloheptenyl. An exemplary multicyclic cycloalkenyl is norbornenyl.
"Cycloalkyl" means a non-aromatic mono- or multicyclic hydrocarbon ring system of 3 to 10 carbon atoms, preferably of 5 to 10 carbon atoms. Preferred ring sizes of rings of the ring system include 5 to 6 ring atoms. Exemplary monocyclic cycloalkyl include cyclopentyl, cyclohexyl, cycloheptyl, and the like. Exemplary multicyclic cycloalkyl include 1-decalin, norbornyl, adamant-(1- or 2-)yl.
"Dialkylamino" means an (alkyl)₂N- group wherein the alkyl groups are independently as herein described.
"Dialkylaminoalkyl" means an (alkyl)₂N-alkyl- group wherein the alkyl groups are independently as herein described.
"Halogeno" refers to fluorine, chlorine, bromine or iodine atom; preferably fluorine and chlorine atom.
As used herein, the term "Heteroaryl" or aromatic heterocycles refers to a 5 to 14, preferably 5 to 10 membered aromatic hetero, mono-, bi- or multicyclic ring. Examples include pyrrolyl, pyridyl, pyrazolyl, thienyl, pyrimidinyl, pyrazinyl, tetrazolyl, indolyl, quinolinyl, purinyl, imidazolyl, thiazolyl, benzothiazolyl, furanyl, benzofuranyl, 1,2,4-thiadiazolyl, isothiazolyl, triazoyl, isoquinolyl, benzothienyl, isobenzofuryl, carbazolyl, benzimidazolyl, isoxazolyl, pyridyl-N-oxide, as well as the fused systems resulting from the condensation with a phenyl group.
As used herein, the terms "Heterocycle" or "Heterocyclic" refer to a saturated, partially unsaturated or unsaturated, non aromatic stable 3 to 14, preferably 5 to 10 membered mono, bi or multicyclic rings wherein at least one member of the ring is a hetero atom. Typically, heteroatoms include, but are not limited to, oxygen, nitrogen, sulphur, selenium, and phosphorus atoms. Preferable heteroatoms are oxygen, nitrogen and sulphur.
Suitable heterocycles are also disclosed in The Handbook of Chemistry and Physics, 76th Edition, CRC Press, Inc., 1995-1996, pages 2-25 to 2-26, the disclosure of which is hereby incorporated by reference.
Preferred non aromatic heterocyclic include, but are not limited to pyrrolidinyl, pyrazolidinyl, imidazolidinyl, oxiranyl, tetrahydrofuranyl, dioxolanyl, dioxanyl, piperidyl, piperazinyl, morpholinyl, pyranyl, imidazolinyl, pyrrolinyl, pyrazolinyl, thiazolidinyl, tetrahydrothiopyranyl, dithianyl, thiomorpholinyl, dihydropyranyl, tetrahydropyranyl, dihydropyranyl, tetrahydro-pyridyl, dihydropyridyl, tetrahydropyrimidinyl, dihydrothiopyranyl, azepanyl, as well as the fused systems resulting from the condensation with a phenyl group.
"Oxoalkyl" means an alkyl where a CH₂ is replaced by a CO wherein alkyl is as defined herein.
"Fused arylheterocyclyl" means a fused aryl and heterocyclyl as defined herein. Preferred fused arylheterocyclyls are those wherein the aryl thereof is phenyl and the heterocyclyl consists of about 5 to about 6 ring atoms. A fused arylheterocyclyl as a variable may be bonded through any atom of the ring system thereof capable of such. The designation of the aza, oxa or thia as a prefix before heterocyclyl portion of the fused arylheterocyclyl define that at least a nitrogen, oxygen or sulphur atom is present respectively as a ring atom. The nitrogen atom of a fused arylheterocyclyl may be a basic nitrogen atom. The nitrogen or sulphur atom of the heterocyclyl portion of the fused arylheterocyclyl may also be optionally oxidized to the corresponding N-oxide, S-oxide or S,S-dioxide. Exemplary preferred fused arylheterocyclyl ring systems include indolinyl, 1,2,3,4-tetrahydroisoquinoline, 1,2,3,4-tetrahydroquinoline, 1H-2,3-dihydro-isoindol-2-yl, 2,3-dihydrobenz[f]isoindol-2-yl, 1,2,3,4-tetrahydrobenz[g]isoquinolin-2-yl.
"Fused arylcycloalkyl" means a fused aryl and cycloalkyl as defined herein. Preferred fused arylcycloalkyls are those wherein the aryl thereof is phenyl and the cycloalkyl consists of about 5 to about 6 ring atoms. A fused arylcycloalkyl as a variable may be bonded through any atom of the ring system thereof capable of such. Exemplary fused arylcycloalkyl includes 1,2,3,4-tetrahydronaphthyl.

As used herein, "pharmaceutically acceptable salts" refer to derivatives of the disclosed compounds wherein the parent compound is modified by making acid or base salts thereof. The pharmaceutically acceptable salts include the conventional non-toxic salts or the quaternary ammonium salts of the parent compound formed, for example, from non-toxic inorganic or organic acids. For example, such conventional non-toxic salts include those derived from inorganic acids such as hydrochloric, hydrobromic, sulfuric, sulfamic, phosphoric, nitric and the like; and the salts prepared from organic acids such as acetic, propanoic, succinic, tartaric, citric, methanesulfonic, benzenesulfonic, glucuronic, glutamic, benzoic, salicylic, toluenesulfonic, oxalic, fumaric, maleic, and the like. Further addition salts include ammonium salts such as tromethamine, meglumine, epolamine, etc., metal salts such as sodium, potassium, calcium, zinc or magnesium. Hydrochloride and oxalate salts are preferred.

The pharmaceutically acceptable salts of the present invention can be synthesized from the parent compound which contains a basic or acidic moiety by conventional chemical methods. Generally, such salts can be prepared by reacting the free acid or base forms of these compounds with a stoichiometric amount of the appropriate base or acid in water or in an organic solvent, or in a mixture of the two. Generally, non-aqueous media like ether, ethyl acetate, ethanol, isopropanol, or acetonitrile are preferred. Lists of suitable salts are found in Remington's Pharmaceutical Sciences, 17th ed., Mack Publishing Company, Easton, PA, 1985, p. 1418, the disclosure of which is hereby incorporated by reference.

"Pharmaceutically" or "pharmaceutically acceptable" refer to molecular entities and compositions that do not produce an adverse, allergic or other untoward reaction when administered to an animal, or a human, as appropriate.

As used herein, "pharmaceutically acceptable carrier" includes any diluents, adjuvants, excipients, or vehicles, such as preserving agents, fillers, disintegrating agents, wetting agents, emulsifying agents, suspending agents, solvents, dispersion media, coatings, antibacterial and antifungal agents, isotonic and absorption delaying agents and the like. The use of such media and agents for pharmaceutical active substances is well known in the art. Except insofar as any conventional media or agent is incompatible with the active ingredient, its use in the therapeutic compositions is contemplated. Supplementary active ingredients can also be incorporated into the compositions.

The compounds of the general formula (A) having geometrical and stereoisomers are also a part of the invention.

Di Ciano et al. (Neuropsychopharmacology 44, 1284-1290, 2019) disclosed that BP1.4979 is a D3 partial agonist with also D2 antagonist property at a low potency.

Indeed, it is active at the cloned human dopamine D3 receptor with a dissociation constant of 1.2 nM and a 30% intrinsic activity when evaluated in a functional test (namely, mitogenesis). In comparison, it displays only a 661 nM Ki value at the human D2 receptor at which it behaves as a pure antagonist. It is, in addition, inactive at 160 other receptors, channels or enzymes.

Now, it has been shown to be unexpectedly effective in alleviating repetitive compulsive behaviours that are illustrative of OCD and tics disorders.

First, targeting D3 pathway is highly challenging because D3 receptors interact with other neurotransmitter systems (like serotonin) involved in OCD.

Also, there is significant interindividual variability in response to medications targeting the dopaminergic system.

Additionally, the use of D3 dopamine receptor partial agonists for OCD treatment has not been considered so far.

Still further, this D3 partial agonist compound was shown to be active in indications in which pure agonists are currently used such as the Restless Leg Syndrome. It was thus surprisingly found to be active in behavioral tests predictive of disorders currently treated by dopamine antagonists.

In any case, the use of this partial D3 agonist is highly advantageous in that it can normalize both excessive and insufficient neurotransmissions contrary to pure agonists and pure antagonists.

This is particularly adapted to dopaminergic signalling in OCD which is very likely not uniform - some brain regions may show dopaminergic hyperactivity while others show hypoactivity.

Altogether, the activity of a selective D3 partial agonist in OCD was thus unexpected.

As far as tics, such as Tourette's syndrome, are concerned, treatments have focused mainly on pure D2 antagonism and there has been uncertainty about the impact on simultaneous D3 activation:
D2 and D3 receptors interactions have been shown in the basal ganglia circuits typically involved in tics and Tourette syndrome. Further, it may be hypothesized that the D3 activation may have a deleterious effect on the D2 blockade.

Altogether, there may be some unpredictability of efficacy on tics with a selective partial D3 agonist.

Additionally, as a selective partial D3 agonist it will involve lesser side effects than a dopamine antagonist, such as tachyphylaxis phenomenon, side effects related to a full receptor blockade.

As used herein, the term « compulsive » refers to the compulsion, i.e., the irresistible urge that a person feels to perform behaviours or mental acts.

As used herein « repetitive » refers to the same behaviours or mental acts that is repeatedly performed by an individual without he is capable to control it.

### Obsessive compulsive and related disorders

They are typically characterized by obsessions and compulsions. They include obsessive compulsive disorder (OCD), and related disorders such as body-focused repetitive behaviour disorder, hoarding disorder, body dysmorphic disorder, trichotillomania disorder, excoriation disorder, olfactory reference disorder.

Obsessions may be defined as unwanted, intrusive, recurrent, and persistent thoughts, urges, or images that cause distressing emotions such as anxiety, fear, or disgust. OCD patients recognize that these thoughts are a product of their mind and that they are excessive or unreasonable. However, the distress that these intrusive thoughts cause cannot be resolved by logic or reasoning. OCD patients try to ease the distress of the obsessional thinking by doing compulsions. Examples of common content of obsessional thoughts include fear of contamination by people or the environment, disturbing sexual thoughts or images, religious, often blasphemous, thoughts or fears, fear of perpetrating aggression or being harmed (self or loved ones), extreme worry something is not complete, extreme concern with order, symmetry, or exactness and fear of losing or discarding something important.

Compulsions may be defined as repetitive behaviours or mental acts that a person feels driven to perform, e.g. in response to an obsession. The behaviours typically prevent or reduce a person's distress related to an obsession temporarily, and they are then more likely to do the same in the future. Compulsions may be excessive responses that are directly related to an obsession (such as excessive hand washing due to the fear of contamination) or actions that are completely unrelated to the obsession. In the most severe cases, a constant repetition of rituals may fill the day, making a normal routine impossible.

Examples of compulsions include excessive or ritualized hand washing or showering, repeated cleaning of household objects, excessively ordering or arranging things in a particular way, repeatedly checking locks, switches, appliances, doors, etc..., frequently seeking approval or reassurance, rituals related to numbers, such as counting, repeating, or doing things a certain number of times (for example, three times), avoiding certain people, places, or situations that cause them distress and trigger obsessions and/or compulsions.

### Body-focused repetitive behaviour disorder

This disorder may be defined as excessively repetitive activities involving the patient's body, such as nail biting, lip biting, or cheek chewing, and repeatedly try to stop the activities.

### Hoarding disorder

This disorder may be defined as the persistent difficulty of getting rid of or parting with possessions, typically due to a perceived need to save the items. Attempts to part with possessions create considerable distress and lead to decisions to save them.

### Body dysmorphic disorder (BDD)

BDD is typically a disorder involving the repetitive patent's perception of flaws in their physical appearance. The perceived flaws are actually not noticeable or appear only slight to other people, but the person with BDD sees them as ugly or abnormal. In other words, people with BDD have distorted body image. BDD is not the same as the typical concerns many people have about their appearance. To be diagnosed as BDD, the preoccupation with perceived defects or flaws in one's appearance must cause significant emotional distress or significant interference in social, academic, occupational, or other important areas of functioning.

Body dysmorphic disorder also involves repetitive behaviours, also known as compulsions or rituals, (such as checking mirrors or seeking reassurance about how one looks, or repetitive thoughts, such as comparing one's appearance with other people).

### Trichotillomania

Trichotillomania, or hair-pulling disorder, consists of repeatedly pulling out one's own hair, most commonly from the scalp, eyebrows, and eyelids. To be diagnosed as trichotillomania, the hair pulling must result in hair loss, the person must have attempted to decrease or stop pulling, and the hair pulling must cause significant distress or significant problems with life functioning.

### Excoriation disorder

A person with excoriation (skin-picking) disorder repeatedly picks at their own skin, to the point of causing skin lesions. Although many people occasionally pick at their skin, skin picking is given a diagnosis of excoriation (skin-picking) disorder when the picking causes skin lesions, the person has repeatedly tried to decrease or stop the picking without success, and the behaviour causes significant distress or significant problems with work, social interactions, or other activities.

### Olfactory reference disorder

Olfactory reference disorder (also known as olfactory reference syndrome) consists of preoccupation with the inaccurate belief that one emits a foul or offensive body odour. In reality, the odour is not detectable or is only slight, but most people with olfactory reference disorder don't realize this. For olfactory reference disorder to be diagnosed, the preoccupation must cause significant distress or significant impairment in functioning -- for example, interference with social interactions, relationships, academics, work, or other important aspects of functioning.

### Tic disorders

Tics refer to rapid, purposeless, repetitive but not rhythmic, involuntary movements (muscle or motor tics) or involuntary, abrupt, often repetitive sounds and/or words (vocal tics). They can be suppressed but only for a short time and only with conscious effort.

There are three major types of tic disorder:
- Provisional tic disorder: children have had motor and/or vocal tics for less than a year;
- Persistent tic disorder (chronic tic disorder): children have had motor or vocal tics (but not both) for more than a year;
- Gilles de la Tourette, also called Tourette syndrome: children have had both motor and vocal tics for more than a year.

Patients with Tourette syndrome may typically suffer from one or more conditions such as attention-deficit/hyperactivity disorder, obsessive-compulsive disorder, anxiety disorder, learning disorders, depression, bipolar disorder, substance use disorder.

They may typically suffer from comorbid OCD. Studies demonstrated that 40 to 60% of the patients diagnosed with the Tourette syndrome also suffer from OCD, and that 5 to 10% of the OCD patients also suffer from the Tourette syndrome.

According to an embodiment, said disorder is obsessive compulsive disorder (OCD).

According to an embodiment said disorder is an obsessive-compulsive related disorder, such as body dysmorphic disorder, skin-picking disorder, hair-pulling disorder/trichotillomania, body-focused repetitive behaviour disorder, hoarding disorder, excoriation disorder and olfactory reference disorder.

Patients suffering from OCD and related disorders typically suffer from various conditions, e.g., conditions for which OCD and related disorders are symptoms thereof, or for which there is a comorbidity with OCD and related disorders.

Typically said patients may suffer from one or more condition selected from generalized anxiety disorder; depressive disorder; eating disorder; tic disorder such as Gilles de la Tourette disorder; body-focused repetitive behaviour; autism; attention-deficit/hyperactivity disorder (ADHD) and obsessive-compulsive personality disorder.

According to an embodiment, said tics disorder is Gilles de la Tourette syndrome.

Patients suffering from tics disorders may typically suffer from various conditions, e.g., conditions for which tics are symptoms thereof, or for which there is a comorbidity with tics disorders.

Typically said patients may suffer from one or more conditions selected from attention-deficit/hyperactivity disorder (ADHD), obsessive-compulsive disorder (OCD), anxiety disorder, learning disorders, depression, bipolar disorder, substance use disorder.

When exerting its activity, the compound of formula (A) may primarily act on the occurrence of compulsions. According to an embodiment, said compound may inhibit and/or decrease the incidence and/or intensity of compulsions.

In the case of OCD and related disorders, the compound may also act on the occurrence of obsessions. According to an embodiment, said compound may inhibit and/or decrease the incidence and/or intensity of obsessions in a patient suffering from OCD and related disorders.

According to another object, the present invention also relates to the above-mentioned therapeutic treatment methods comprising the administration of a compound according to the invention together with a pharmaceutically acceptable carrier or excipient to a patient in the need thereof.

According to a further object, the present invention also relates to combinations comprising a compound of the invention and one or more further active ingredient(s).

The identification of those patients i.e., subjects who are in need of treatment of herein-described disorders is well within the ability and knowledge of one skilled in the art. A clinician skilled in the art can readily identify, by the use of clinical tests, physical examination, genetic tests and medical/family history, those subjects who are in need of such treatment.

A therapeutically effective amount can be readily determined by the attending diagnostician, as one skilled in the art, by the use of conventional techniques and by observing results obtained under analogous circumstances. In determining the therapeutically effective amount, a number of factors are considered by the attending diagnostician, including, but not limited to: the species of subject; its size, age, and general health; the specific disease involved; the degree of involvement or the severity of the disease; the response of the individual subject; the particular compound administered; the mode of administration; the bioavailability characteristic of the preparation administered; the dose regimen selected; the use of concomitant medication; and other relevant circumstances.

The amount of a compound of formula (A), which is required to achieve the desired biological effect, will vary depending upon a number of factors, including the dosage of the drug to be administered, the chemical characteristics (e.g., hydrophobicity) of the compounds employed, the potency of the compounds, the type of disease, the diseased state of the patient and the route of administration.

In the context of the invention, the term "treating" or "treatment", as used herein, means reversing, alleviating, inhibiting the progress of, or preventing the disorder to which such term applies, or one or more symptoms of such disorder or condition.

"Therapeutically effective amount" means an amount of a compound/ medicament according to the present invention effective in producing the desired therapeutic effect.

According to the invention, the term "patient", or "patient in need thereof", is intended for a human or non-human mammal affected or likely to be affected with a neuropsychological disorder. Preferably, the patient is a human.

Typical dose ranges are from 10 mg to 50 mg once, twice or three times a day, preferably 15 to 30 mg two or three times a day. The preferred dosage of drug to be administered is likely to depend on such variables as the type and extent of progression of the disease or disorder, the overall health status of the particular patient, the relative biological efficacy of the compound selected, and formulation of the compound excipient, and its route of administration.

The compounds of the present invention are capable of being administered in unit dose forms, wherein the term "unit dose" means a single dose which is capable of being administered to a patient, and which can be readily handled and packaged, remaining as a physically and chemically stable unit dose comprising either the active compound itself, or as a pharmaceutically acceptable composition, as described hereinafter.

Compounds provided herein can be formulated into pharmaceutical compositions by admixture with one or more pharmaceutically acceptable excipients. Such compositions may be prepared for use in oral administration, particularly in the form of tablets or capsules; or parenteral administration, particularly in the form of liquid solutions, suspensions or emulsions; or intranasally, particularly in the form of powders, nasal drops, or aerosols; or dermally, for example, topically or via trans-dermal patches.

The compositions may conveniently be administered in unit dosage form and may be prepared by any of the methods well known in the pharmaceutical art, for example, as described in Remington: The Science and Practice of Pharmacy, 20th ed.; Gennaro, A. R., Ed.; Lippincott Williams & Wilkins: Philadelphia, PA, 2000. Pharmaceutically compatible binding agents and/or adjuvant materials can be included as part of the composition. Oral compositions will generally include an inert diluent carrier or an edible carrier.

The tablets, pills, powders, capsules, troches and the like can contain one or more of any of the following ingredients, or compounds of a similar nature: a binder such as microcrystalline cellulose, or gum tragacanth; a diluent such as starch or lactose; a disintegrant such as starch and cellulose derivatives; a lubricant such as magnesium stearate; a glidant such as colloidal silicon dioxide; a sweetening agent such as sucrose or saccharin; or a flavouring agent such as peppermint, or methyl salicylate. Capsules can be in the form of a hard capsule or soft capsule, which are generally made from gelatine blends optionally blended with plasticizers, as well as a starch capsule. In addition, dosage unit forms can contain various other materials that modify the physical form of the dosage unit, for example, coatings of sugar, shellac, or enteric agents. Other oral dosage forms syrup or elixir may contain sweetening agents, preservatives, dyes, colourings, and flavourings. In addition, the active compounds may be incorporated into fast dissolve, modified-release or sustained-release preparations and formulations, and wherein such sustained-release formulations are preferably bi-modal.

Preferred formulations include pharmaceutical compositions in which a compound of the present invention is formulated for oral or parenteral administration, or more preferably those in which a compound of the present invention is formulated as a tablet.

Liquid preparations for administration include sterile aqueous or non-aqueous solutions, suspensions, and emulsions. The liquid compositions may also include binders, buffers, preservatives, chelating agents, sweetening, flavouring and colouring agents, and the like. Non-aqueous solvents include alcohols, propylene glycol, polyethylene glycol, vegetable oils such as olive oil, and organic esters such as ethyl oleate. Aqueous carriers include mixtures of alcohols and water, buffered media, and saline. In particular, biocompatible, biodegradable lactide polymer, lactide/glycolide copolymer, or polyoxyethylene-polyoxypropylene copolymers may be useful excipients to control the release of the active compounds. Intravenous vehicles can include fluid and nutrient replenishers, electrolyte replenishers, such as those based on Ringer's dextrose, and the like. Other potentially useful parenteral delivery systems for these active compounds include ethylene-vinyl acetate copolymer particles, osmotic pumps, implantable infusion systems, and liposomes.

In general terms, the compounds of this invention may be provided in an aqueous physiological buffer solution containing 0.1 to 10% w/v compound for parenteral administration.

Preferred tablets contain lactose, cornstarch, magnesium silicate, croscarmellose sodium, povidone, magnesium stearate, or talc in any combination. It is also an aspect of the present disclosure that a compound of the present invention may be incorporated into a food product or a liquid.

Alternative modes of administration include formulations for inhalation, which include such means as dry powder, aerosol, or drops. They may be aqueous solutions containing, for example, polyoxyethylene-9-lauryl ether, glycocholate and deoxycholate, or oily solutions for administration in the form of nasal drops, or as a gel to be applied intranasally. Formulations for buccal administration include, for example, lozenges or pastilles and may also include a flavoured base, such as sucrose or acacia, and other excipients such as glycocholate. Formulations suitable for rectal administration are preferably presented as unit-dose suppositories, with a solid based carrier, such as cocoa butter, and may include a salicylate. Formulations for topical application to the skin preferably take the form of an ointment, cream, lotion, paste, gel, spray, aerosol, or oil. Carriers which can be used include petroleum jelly, lanolin, polyethylene glycols, alcohols, or their combinations. Formulations suitable for transdermal administration can be presented as discrete patches and can be lipophilic emulsions or buffered, aqueous solutions, dissolved and/or dispersed in a polymer or an adhesive.

The following examples illustrate the invention, but do not limit it. The starting products used are products which are known or prepared using known methods.

Unless otherwise stated, percentages are weight percentages.

### Figures

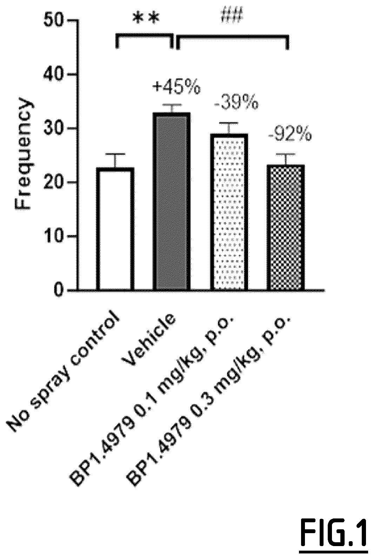

Figure 1 illustrates the results obtained by the test described in example 1.

### Examples

The activity of compound BP1.4979 on OCD was investigated *in vivo* on a mouse model for obsessive-compulsive disorder derived from the one of Chen et al Behav. Brain. Funct. (2021), 17:4. In this model the spray elicits a repetitive behaviour and is considered as a proxy for OCD. Briefly, mice where were gently sprayed with a 2% sucrose spray, and the subsequent grooming behaviour of the mice was recorded for 10 minutes.

Mice were divided into the following groups (22 mice per group):
- Control group: mice were not subjected to spray
- Vehicle group: mice were administered p.o. the vehicle 1h before receiving a 2% sucrose spray
- Treatment group 1: mice were administered p.o. the BP1.4979 at the dose of 0.1 mg/kg 1h before receiving a 2% sucrose spray
- Treatment group 2: mice were administered p.o. the BP1.4979 at the dose of 0.3 mg/kg 1h before receiving a 2% sucrose spray.

The grooming frequency was analyzed by video tracking over 10 min post-spray.

Results are illustrated in Figure 1 where **, ##: p<0.01, One way ANOVA followed by Dunnett's test.

Figure 1 shows that BP1.4979 significantly reduces the grooming frequency at both doses (0.1 and 0.3 mg/kg), compared to vehicle-treated animals. Interestingly, BP1.4979 nearly fully inhibits the grooming frequency at its higher dose.

## Claims

1. A compound of formula: 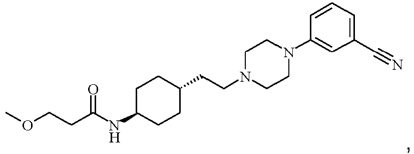
*N*-(4-{2-[4-(3-Cyanophenyl)piperazin-1-yl]ethyl}cyclohexyl)-3-methoxypropanamide or a pharmaceutically acceptable salt thereof, or its hydrates, or hydrated salts, or the polymorphic, crystalline structures thereof,
for use for treating or preventing a repetitive compulsive disorder chosen from obsessive compulsive disorder (OCD) and related disorders, and tic disorders in a patient.

2. The compound for use according to claim 1 wherein said disorder is obsessive compulsive disorder (OCD).

3. The compound for use according to claim 1 wherein said obsessive compulsive related disorders are chosen from body dysmorphic disorder, skin-picking disorder, hair-pulling disorder/trichotillomania, body-focused repetitive behaviour disorder, hoarding disorder and olfactory reference disorder.

4. The compound for use according to claim 1 wherein said disorder is Gilles de la Tourette syndrome.

5. The compound for use according to anyone of the preceding claims suitable for inhibiting and/or decreasing the incidence and/or intensity of compulsions.

6. The compound for use according to anyone of claims 2 or 3 for inhibiting and/or decreasing the incidence and/or intensity of obsessions.

7. The compound for use according to anyone claims 2 or 3 wherein said patient suffers from one or more condition selected from generalized anxiety disorder; depressive disorder; eating disorder; tic disorder such as Gilles de la Tourette disorder; body-focused repetitive behaviour; autism; attention-deficit/hyperactivity disorder (ADHD) and obsessive-compulsive personality disorder.

8. The compound for use according to claim 4 wherein said patient suffers from one or more condition selected from attention-deficit/hyperactivity disorder, obsessive-compulsive disorder, anxiety disorder, learning disorders, depression, bipolar disorder, substance use disorder.

9. The compound for use according to anyone of the preceding claims wherein it is administered at a dose comprised between 10 mg and 50 mg once, twice or three times a day.
